# EUROPEAN PATENT SPECIFICATION

(11) **EP 3 936 634 B1**
(45) Date of publication and mention of the grant of the patent: **13.09.2023**
(21) Application number: 19918349.2
(22) Date of filing: 14.11.2019
(51) Int. Cl.: C23C 8/10, C23C 8/02, C23C 8/80, A61L 27/04, A61L 27/30, C22C 16/00, C23C 8/12

(54) **PREPARATION METHOD OF A MEDICAL IMPLANT BY APPLICATION OF OXIDIZED CERAMIC LAYER ON SURFACE OF ZIRCONIUM**
HERSTELLUNGSVERFAHREN EINER MEDIZINISCHEN PROTHESE DURCH ANWENDUNG EINER OXIDIERTEN KERAMISCHEN SCHICHT AUF DER OBERFLÄCHE VON ZIRKONIUM
PROCÉDÉ DE PRÉPARATION D'UN IMPLANT MÉDICAL PAR APPLICATION D'UNE COUCHE CÉRAMIQUE OXYDÉE SUR UNE SURFACE DE ZIRCONIUM

(30) Priority: 07.03.2019 CN 201910173412
(43) Date of publication of application: 12.01.2022
(73) Proprietor: Suzhou Microport Orthorecon Co., Ltd., Suzhou, Jiangsu 215000 (CN)
(72) Inventor: SHEN, Yu, Suzhou, Jiangsu 215000 (CN); YU, Tianbai, Suzhou, Jiangsu 215000 (CN); WENG, Chih-Hsin, Suzhou, Jiangsu 215000 (CN)
(74) Representative: Patentship Patentanwaltsgesellschaft mbH
(86) International application number: PCT/CN2019/118460
(87) International publication number: WO 2020/177386

(56) References cited:
- WO-A1-98/42390
- CN-A- 1 380 856
- CN-A- 1 553 967
- CN-A- 104 818 409
- CN-A- 107 142 444
- CN-A- 107 675 122
- CN-A- 107 675 122
- CN-A- 109 706 421
- US-A1- 2002 042 656
- US-A1- 2007 137 734
- US-A1- 2007 251 604
- US-A1- 2010 074 789

## Description

### TECHNICAL FIELD

The present application pertains to the field of materials for use in medical implants, and relates in particular to a preparation method and application of an oxidized ceramic layer on the surface of zirconium and zirconium alloy.

### BACKGROUND

As have been confirmed by scientists many years ago, zirconium and zirconium alloys have excellent mechanical properties, corrosion resistance and biocompatibility. Therefore, they have been recognized as very good materials for use in medical implants. For the materials used for medical implants, service life of the medical implant is most important. Especially, when the medical implant is used in the young patient, the desirable implant is able to be used throughout the lifetime of the patient.

However, zirconium and zirconium alloys are not sufficiently resistant to wear, which limits their application to bearing implants such as hip and knee joint replacements. The service lifespan of such bearing implants are affected by the factors including not only mechanical properties, corrosion resistance and biocompatibility of the used zirconium or zirconium alloy but also the generated wear of zirconium or zirconium alloy surface as it moves relative to another contacting surface made of, such as ultra-high molecular weight polyethylene. Such wear not only create numerous wear particles to enhance friction coefficients of articular surfaces and thus further intensify wear, but also release metal ions that may cause long-lasting harm to human health. Therefore, it is necessary to strengthen wear resistance property when zirconium or a zirconium alloy is used manufacture such bearing types of implant devices.

U.S. Patent US2987352A discloses to oxidize a zirconium/zirconium alloy in air to form a blue-black oxide layer on their surfaces. The formed blue-black oxidation ceramic surface layer is very hard and compact, imparting significantly improved wear resistance to the zirconium/zirconium alloy. Following this thought, U.S. Patent US5037438A discloses a zirconium alloy prosthesis with a zirconium oxide surface. However, the oxidation ceramic surface layer generally has a non-uniform thickness. In order to decrease friction coefficients of relative movement surfaces to alleviate wear, it is necessary to reduce the roughness of the surface oxidation ceramic layer as much as possible. Therefore, the oxide surface layer must be finely polished. However, the polishing process results in various thickness losses of the oxide surface layer at different locations. Besides, the blue-black oxidation ceramic surface layer tends to have a very thin and uneven thickness. This causes the complete removal of the oxidation ceramic surface layer at locations with thinner thicknesses, and thus results in the loss of integrity of the oxidation ceramic surface layer, which seriously impairs wear resistance of the prosthesis. The uneven oxidation ceramic surface layer results in an uneven contact with the metal substrate at the interface, which weakens bonding strength between the oxidation ceramic surface layer and the metal substrate. This causes an easy peel-off of the oxidation ceramic surface layer from the metal substrate during use, and thus brings great risks.

Chinese Patent Application, CN 107675122 A, discloses the preparation method of the non-stoichiometric ratio oxidation film on the surface of a zirconium-niobium alloy comprising sequentially grinding and polishing the surface of the zirconium-niobium alloy material, and forming the non-stoichiometric ratio oxidation film on the surface of the zirconium-niobium alloy by carrying out the specific oxidation treatment. However, the oxidation film obtained by this method has a surface roughness that does not meet the medical implant criterion, which has to be subjected to a secondary polishing after the oxidation treatment. Therefore, it cannot solve the problem that the oxide film is locally too thin or even damaged caused by the secondary polishing.

U.S. Patent Application, US 2007/0251604 A1, discloses a method of reworking a zirconium or zirconium alloy comprising an oxidation and/or nitridation layer on its surface, comprising treating the zirconium or zirconium alloy under reduced pressure or in an inert gas environment at an elevated temperature until the oxidation and/or nitridation layer is substantially removed from the surface. Such a method is directed to reclaim the rejected components due to the failure of the oxidation treatment to carry out the secondary oxidation treatment, and does not teach how to achieve a uniform and unbroken oxide layer.

U.S. Patent Application, US 2007/0137734 A1, discloses a medical implant of zirconium or zirconium alloy comprising a thick diffusion hardened zone and a ceramic layer, as well as a method of treating the medical implant in vacuum or in an inert gas environment at an elevated temperature. Such a method is able to generate a specific diffusion hardened zone, but is unable to solve problems created by the secondary polishing after the oxidation treatment.

U.S. Patent Application, US 2002/0042656 A1, discloses a method of making a coating of a blue-black or black oxidized zirconium of uniform thickness on a zirconium or zirconium alloy material, comprising the step of changing the surface roughness of the zirconium or zirconium alloy and the subsequent step of the oxidation treatment. However, such a method cannot solve problems created by the secondary polishing after the oxidation treatment.

The international PCT Patent Application, WO 98/42390, discloses a method of forming a oxide coating on zirconium or zirconium alloys, comprising changing the surface roughness of the zirconium or zirconium alloy, and generating the oxide coating of a uniform thickness via the oxidation treatment. However, such a method cannot solve problems created by the secondary polishing after the oxidation treatment. Further studies have shown that the growth of a zirconium oxide coating proceeds at a rate that is not constant and will experience a turning point. Before this point, increase of thickness for the coating follows the parabolic profile and the formed oxidation ceramic surface layer has an excellent compactness, a high bonding strength and a good wear resistance. After this point, increase of thickness for the coating follows the linear profile, and the formed coating has numerous micro-cracks therein, a poor bonding strength and a bad wear resistance (Tatsumi Arima et al. , Oxidation properties of Zr-Nb alloys at 973-1273 K in air. Progress in Nuclear Energy, 51(2009):307-312.). For an oxidation ceramic surface layer, an optimal thickness is in the range of 3-7 µm. With the thickness in this range, the coating possesses an optimal internal compactness, a high bonding strength and an excellent wear resistance. However, in practical operations, after a formed oxidation ceramic surface layer is polished to a target surface roughness Ra of 0.02 µm or less required for a medical implant, the oxidation ceramic surface layer lose a thickness of 2-5 µm. Obviously, if the oxidation ceramic surface layer having a thickness within the optimal thickness range has been polished, the remaining oxidation ceramic surface layer would have an ultimate thickness that cannot meet application requirements. Whereas, in order to obtain a sufficient remaining thickness of the oxidation ceramic surface layer after the polishing process, it is required to form an initial oxidation ceramic surface layer with an increased thickness. However, the increased thickness impairs properties of the oxidation ceramic surface layer.

Additionally, polishing is indispensible in traditional surface oxidation process. If the cooling process after the completion of high-temperature oxidation is a slow cooling process, the oxidation ceramic surface layer has its thickness further increased in this cooling process as the zirconium or a zirconium alloy is still exposed in the oxidizing atmosphere. However, the oxide layer formed at improper temperatures has a poor quality and has to be polished and removed. If the cooling process is a quick cooling process, due to the significant temperature difference between the inside and outside of the workpiece, thermal stress causes micro-cracks to develop in an outermost surface area of the oxide layer easily. This also results in the necessity of polishing and removal of such area.

In view of the above cases, it is desirable to develop a new method for controlling the roughness of the oxidation ceramic surface layer to avoid the adverse effects of the polishing process on the oxidation ceramic surface layer.

### SUMMARY

In order to overcome the above problems, present application proposes a method for preparing a medical implant, comprising forming an oxidation ceramic layer on a surface of zirconium or a zirconium alloy, which is able to directly obtain the oxidation ceramic surface layer having a satisfied roughness and a controlled uniform thickness without the need of a polishing process after the surface oxidization treatment on the zirconium or zirconium alloy, and thus enables to solve the problems of the surfaces of the zirconium or zirconium alloys caused by polishing of the oxidation ceramic surface layer. Here, the term of "zirconium/zirconium alloy" refers to that the method for preparing an oxidation ceramic surface layer is applicable to pure zirconium metal and zirconium alloys.

In order to solve the above problems, present application provides a method for preparing a medical implant, comprising forming an oxidation ceramic layer on a surface of zirconium or a zirconium alloy, the formation of the oxidation ceramic layer on a surface of zirconium or a zirconium alloy comprising steps of:
1) lowering a roughness of a targeted area of the zirconium or zirconium alloy on which a surface oxidization treatment is to be performed, the roughness being controlled to 0.01 µm or less;
2) performing the surface oxidation treatment by heating to a temperature in the range of 500°C to 700 °C and maintaining the temperature for 0.5-10 h in an atmosphere containing an oxidizing gas to form an oxidation ceramic surface layer; and
3) introducing an inactive gas to replace the oxidizing gas used in step 2) and initiating a cooling process to complete preparation of the oxidation ceramic surface layer without performing a subsequent treatment of lowering a roughness of the oxidation ceramic surface layer.

Optionally, the roughness is lowered to a value in the range of 0.002µm to 0.010 µm in step 1).

Optionally, after the oxidation treatment in step 2), a thickness of the oxidation ceramic surface layer on the targeted area is controlled within a range of 1 µm to 20 µm.

Optionally, the roughness of the targeted area is lowered in step 1) by means of grinding, mechanical polishing, fine machining, vibratory polishing or any combination thereof.

Optionally, the inactive gas in step 3) is one or more selected from the group consisting of nitrogen and rare gases.

Optionally, the targeted area for the surface oxidization treatment is an area to be brought into direct contact with another bearing surface.

Optionally, the atmosphere containing the oxidizing gas in step 2) is an atmosphere containing oxygen and/or ozone.

According to present application, roughness of a surface has been controlled before the surface oxidization treatment, thereby enabling the surface roughness after oxidization treatment to meet application requirements directly. Moreover, the replacement of oxidizing gas with the inactive gas after the surface oxidization treatment avoids the formation of poor-quality layer that has to be removed during the cooling process. The above two advantages enables to completely avoid the necessity of subsequent polishing process, and to remain integrity and uniformity of the oxidation ceramic surface layer as well as ensuring the protective property of the oxidation ceramic surface layer.

### BRIEF DESCRIPTION OF THE DRAWINGS

Fig. 1 is a schematic cross-sectional view illustrating the structure of a material for use in medical implants according to particular embodiments.
Fig. 2 is a metallurgical microscope image showing cross section of Sample 1 that has been oxidized according to Embodiment 1.
Fig. 3 is a metallurgical microscope image showing cross section of the sample of Fig. 2 that has been ground and polished.
Fig. 4 is a metallurgical microscope image showing cross section of Sample 2 that has been oxidized according to Embodiment 1.
Fig. 5 is a metallurgical microscope image showing cross section of Sample 3 that has been oxidized according to Embodiment 2.
Fig. 6 is a metallurgical microscope image showing cross section of the sample of Fig. 5 that has been ground and polished.
Fig. 7 is a metallurgical microscope image showing cross section of Sample 4 that has been oxidized according to Embodiment 2.
Fig. 8 is a metallurgical microscope image showing cross section of Sample 5 that has been oxidized according to Embodiment 3.
Fig. 9 is a metallurgical microscope image showing cross section of Sample 6 that has been oxidized according to Embodiment 3.

In the figures,
10, oxidation ceramic surface layer; 20, oxygen-rich diffusion layer; 30, metal substrate.

### DETAILED DESCRIPTION

The method for preparing an oxidation ceramic layer on a surface of zirconium or a zirconium alloy comprises the following specific steps:
(1) Lowering roughness Ra of the zirconium/zirconium alloy surface on which the surface oxidization treatment is to be performed, at least the portion thereof to be brought into direct contact with another bearing surface to a value below 0.01 µm (i.e., Ra<0.01 µm), preferably in the range of 0.002-0.010 µm (i.e., Ra=0.002-0.010 µm), more preferably in the range of 0.003-0.008 µm (i.e., Ra=0.003-0.008 µm). For example, Ra may be lowered into the range of 0.004-0.007 µm (i.e., Ra=0.004-0.007 µm) or of 0.005-0.006 µm (i.e., Ra=0.005-0.006 µm). Here, the word "bearing" means the relationship that the surface of the implant is to be squeezed against and moves relative to another object (another implant or human bone, etc.), such as the relationship between the tibial joint surface of knee joint and the femoral joint surface. The term of "another bearing surface" refers to the surface of another implant or human bone to be brought into contact with the implant surface that has been performed a surface oxidization treatment.

The common zirconium or zirconium alloy medical implants that have been performed surface oxidization treatments, in particular hip or knee joint prostheses fabricated from zirconium-niobium alloys, are generally required to have a surface roughness of 0.02 µm or less. Such surface roughness has to be realized by polishing the oxidation ceramic surface layer after oxidation treatment in prior art. On the contrary, according to present application, the metal surface is pre-treated to control the roughness within a predetermined range in advance. Inventors have confirmed through repeat experiments that the metal having the roughness within the predetermined range is able to be oxidized and thus form an oxidation ceramic surface layer having a surface roughness that meets application requirements directly, enabling to omit the polishing step after the oxidization treatment. In this surface roughness control step, if the roughness Ra is lowered to a value above 0.010 µm, the oxidation ceramic layer resulting from the surface oxidation treatment may have a surface roughness Ra greater than 0.02 µm that fails to satisfy requirements; and if the roughness Ra is lowered to 0.002 µm or less, the cost is too high and the roughness exceed actual needs, which is thus not recommended in consideration of cost effectiveness

The surface roughness modification can be accomplished by grinding, mechanical polishing, fine machining, vibratory polishing or any combination thereof.
(2) Performing an surface oxidation treatment to the zirconium or zirconium alloy that has the surface roughness thereof changed in step (1). Specifically, the zirconium or zirconium alloy from step (1) is placed in an atmosphere containing an oxidizing gas, heated to a temperature in the range of 500-700°C (Preferably, 550-600°C) and maintained the zirconium or zirconium alloy at the high temperature for 0.5-10 h (Preferably, 2-6 h). The oxidation ceramic surface layer formed on the zirconium or zirconium alloy has a thickness of 1-20 µm, preferably 3-10 µm, more preferably 3-7 µm, e.g., 4 µm, 5 µm or 6 µm,. The oxidizing gas refers to the gas having an oxidizability, such as oxygen or ozone, and the atmosphere containing the oxidizing gas refers to the atmosphere that contains said oxidizing gas and is preferred to be air in consideration of economy and easy availability.

If the oxidation ceramic surface layer grows to have an excessively large thickness, the occurrence of numerous micro-cracks in the layerc as well as the poor bonding strength and bad wear resistance are resulted. If the layer is too thin, it may suffer from insufficient wear resistance. If the oxidation ceramic surface layer has a thickness in the above range, the layer is able to have an optimal internal compactness, a high bonding strength and an excellent wear resistance. The surface oxidation ceramic layer does not need to be too thick as there is no need to polish the layer in subsequent. Therefore, the thickness of surface oxidation ceramic layer can be controlled within the above range directly and the elimination of subsequent polishing enables to prove integrity and uniformity of the oxidation ceramic surface layer, resulting in additional improvements in its overall performance.

Preferably, during a cooling process, the oxidizing gas may be replaced by introducing nitrogen or an inert gas such as helium, neon or argon, or any combination thereof. In this way, surface oxidation treatment of the zirconium/zirconium alloy is terminated and the temperature slowly declines to room temperature.

The subsequent replacement with the inactive gas avoids the formation during the cooling process of a membrane layercoating with inferior quality that has to be removed in subsequent, thus resulting in a further increase in the quality of the oxidation ceramic surface layer. Through the upstream roughness control and the downstream gas replacement, the polishing after oxidization treatment is successfully omitted and thus the problems arising from the polishing are solved.

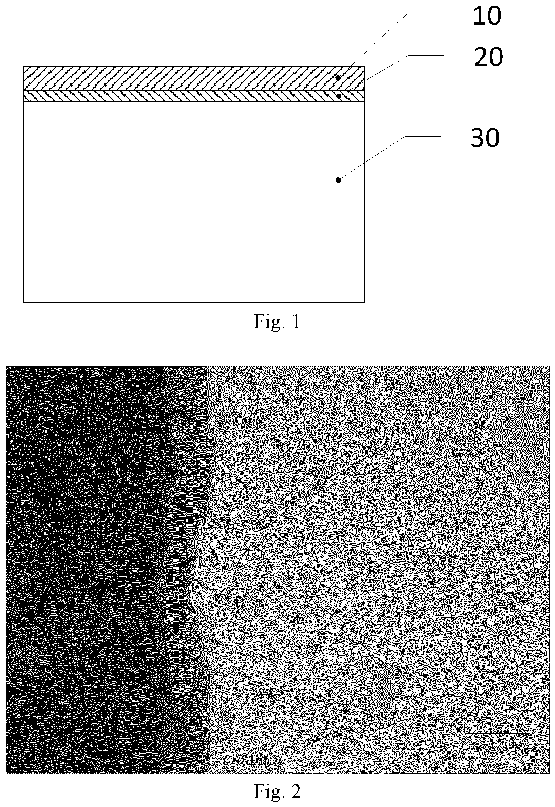

The above method can be used to fabricate a material for use in medical implants, which includes a metal substrate 30, a oxygen-rich diffusion layer 20 and a oxidation ceramic surface layer 10 that are arranged from inside to outside, as shown in Fig. 1. The metal substrate 30 is a metallic material that has not experienced a surface oxidation treatment. The oxidation ceramic layer 10 is a layer in which oxygen element is present essentially in the form of the oxide. The oxygen-rich diffusion layer 20 is a layer in which the content of oxygen is higher than the content of oxygen in metal substrate and the oxygen element is present essentially in the form of solute atoms. Here, the metal substrate 30 is zirconium metal or a zirconium alloy, and the oxygen-rich diffusion layer 20 and the oxidation ceramic surface layer 10 are formed by the above method. Such material is suitable for use in the fabrication of medical implants, in particular bearing implants in hip or knee joints, which are required to have bearing performances.

It is to be noted that in each of the metallurgical microscope photographs shown in Figs. 2 to 9, the middle dark region is an oxidation ceramic surface layer 10, and the rightmost brightest region is a metal substrate 30. Limited by the ability of the used metallurgical microscope, it would be difficult to distinguish an oxygen-rich diffusion layer 20 from a metal substrate 30 in the photos. In fact, the oxygen-rich diffusion layer 20 is a light-colored layer having a thickness of 1 to 2-µm and close to the dark-colored oxidation ceramic surface layer 10.

For ease of understanding, the method for preparing an oxidation ceramic layer on a surface of zirconium or a zirconium alloy will be further described below with reference to several embodiments. These embodiments are presented for the mere purpose of illustrating the present application and do not limit protection scope thereof in any sense.

Unless particularly noted, each material or reagent used in the following embodiments is commercially available, and each process or parameter adopted in the method can be realized by conventional techniques.

### Embodiment 1

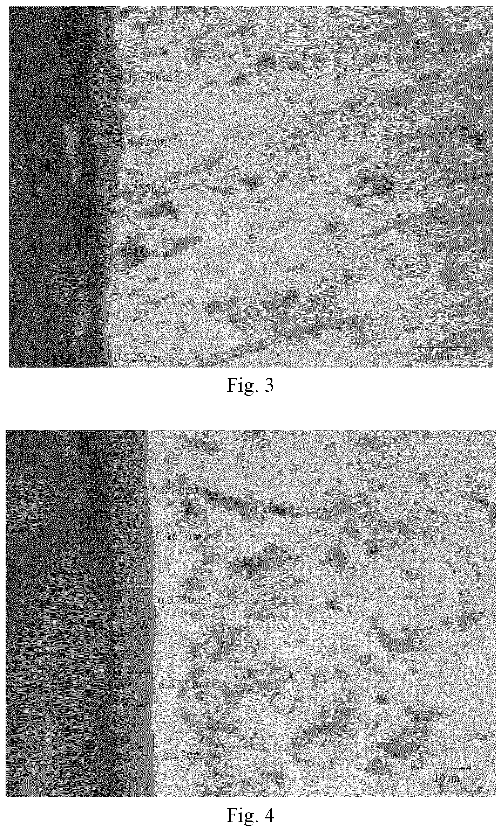

Zr-2.5Nb alloy (a zirconium alloy containing 2.5 wt% of Nb) samples were ground and polished so that Sample 1 had a lowered surface roughness Ra of 0.3792 µm and Sample 2 had a lowered surface roughness Ra of 0.0038 µm. The samples 1 and 2 were together placed into a tube furnace, heated to 550 °C in air and maintained at the temperature for 6 h. Subsequently, air was evacuated and argon was introduced to the furnace. After the slow cooling at a rate of 5 °C/min to 100 °C or below, the samples 1 and 2 were taken out and measured with a roughness gauge. The results showed, after the oxidation treatment, the roughness Ra of Sample 1 increased to 0.6852 µm and that of Sample 2 increased to 0.0158 µm. Fig. 2 is a metallurgical microscopic image of a cross section of Sample 1 that has been oxidized, which shows a metal substrate (right), an oxidation ceramic surface layer (left) and an oxygen-rich diffusion layer between the metal substrate and the oxidation ceramic surface layer. At this time, a cross section image of Sample 1 after the oxidation treatment was taken and shown in Fig. 3. Since the surface roughness Ra of Sample 2 after the oxidation treatment has already met the requirement of Ra < 0.02 µm, there is no need to perform a polishing treatment. The cross section image of Sample 2 after the oxidation treatment was taken by a metallurgical microscope and shown in Fig. 4.

As can be seen from Fig. 2, Sample 1 that has experienced surface oxidation treatment possesses an oxidation ceramic surface layer having an average thickness of about 5.82 µm. Such thickness falls in the optimal thickness range of 3-7 µm. However, as the surface of sample 1 before oxidation treatment fluctuates seriously, the interface between the oxidation ceramic surface layer and the substrate fluctuates seriously. In addition, the growth of the oxidation ceramic surface layer was accompanied by the increase of surface roughness. The above two factors together results in the sharp increase of surface roughness of the oxidation ceramic surface layer. Fig. 3 is a metallurgical microscope image showing the cross section of Sample 1 that has been further ground and polished to a Ra of 0.0184 µm. As can be seen, after being ground and polished, the remaining oxidation ceramic surface layer exhibits an extremely uneven distribution of thickness, even the exposure of zirconium-niobium alloy substrate at partial area. The reason for this was that during the grinding and polishing process for a low roughness, the convex portions of the oxidation ceramic surface layer experiences a large thickness loss while the concave portions thereof experiences few thickness loss. After the grinding and polishing treatment, the oxidation ceramic surface layer has a thickness of 4.728 µm at the thickest location but almost no reservation of the oxidation ceramic layer at the thinnest location. Apparently, this oxidation ceramic surface layer is unusable.

By contrast, Sample 2 of Fig. 4 had an oxidation ceramic surface layer with a thickness of about 6.21 µm within the optimal thickness range of 3-7 µm. Since the initial surface roughness of Sample 2 is very low, the formed oxidation ceramic surface layer has a uniform thickness varying from 5.859 µm to 6.373 µm, i.e., having a tolerance of only 0.26 µm. Since this oxidation ceramic surface layer was totally satisfactory in terms of surface roughness, it is unnecessary to perform a further polishing treatment and integrity of the layer would not be impaired, so that the oxidation ceramic surface layer is able to possess a great improvement in property. The oxidation ceramic surface layer obtained by the present method has a uniform thickness, in which the interface between the surface oxidation ceramic layer and the substrate is substantially a horizontal plane, making it an ideal oxidation ceramic surface layer of a zirconium/zirconium alloy.

### Embodiment 2

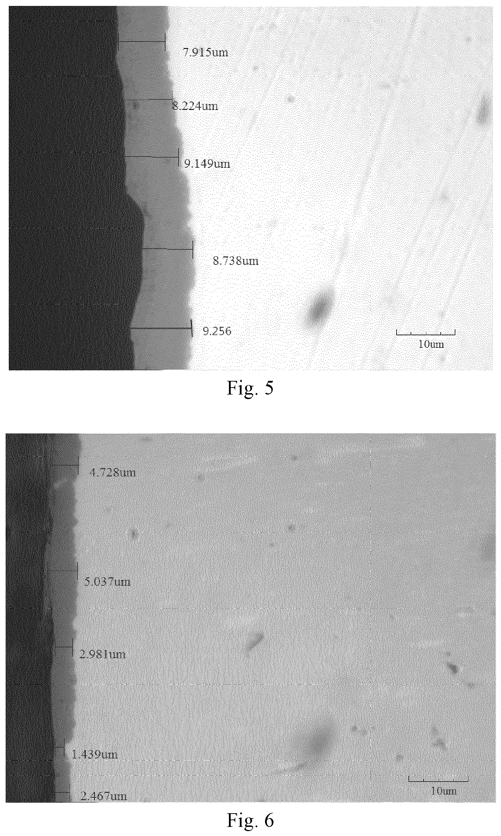

Industrial pure zirconium were ground and polished so that Sample 3 had a lowered surface roughness Ra of 0.3886 µm and Sample 4 had a lowered surface roughness Ra of 0.0042 µm. Samples 3 and 4 were together placed into a tube furnace, heated to 600 °C in air and maintained at the temperature for 4 h. Subsequently, air was evacuated and argon was introduced to the furnace. After the slow cooling at a rate of 5 °C/min to 100 °C or below, the samples 3 and 4 were taken out and measured with a roughness gauge. The results showed, after the oxidation treatment, the roughness Ra of Sample 3 increased to 0.7705 µm and that of Sample 4 increased to 0.0158 µm. Fig. 5 is a metallurgical microscopic image showing a cross section of Sample 3 that has been oxidized, while Fig. 6 is a metallurgical microscopic image showing a cross section of Sample 3 that has been further ground and polished to a surface roughness Ra of 0.0177 µm. Since the surface roughness Ra of Sample 4 after the oxidation treatment has already met the requirement of Ra < 0.02 µm, there is no need to perform a polishing treatment. The cross section image of Sample 4 after the oxidation treatment was taken by a metallurgical microscope and shown in Fig. 4.

As shown in Fig. 5, Sample 3 that has experienced surface oxidation treatment possesses an oxidation ceramic surface layer having an average thickness of about 8.66 µm. The surface of Sample 3 fluctuates seriously and the interface between the oxidation ceramic surface layer and substrate in Sample 3 is a curved interface. Fig. 6 is a metallurgical microscope image showing the cross section of Sample 3 that has been further ground and polished to a Ra of 0.0177 µm. After the grinding and polishing treatment, the oxidation ceramic surface layer remains a thickness of 5.037 µm at the thickest location and a thickness of merely 1.439 µm at the thinnest location. Although the ground and polished oxidation ceramic surface layer still covers the entire surface of Sample No. 3, the remaining oxidation ceramic surface layer has an uneven thickness and a poor protective performance at relatively thin locations. Notably, in order to ensure total coverage of the oxidation ceramic surface layer after the grinding and polishing treatment, the surface oxidation treatment is required to prepare a more thick oxidation ceramic surface layer. However, the oxidation ceramic surface layer formed under the above roughness has already exceeded the optimal thickness range of 3-7 µm, which affects the performance of the surface oxidation ceramic layer.

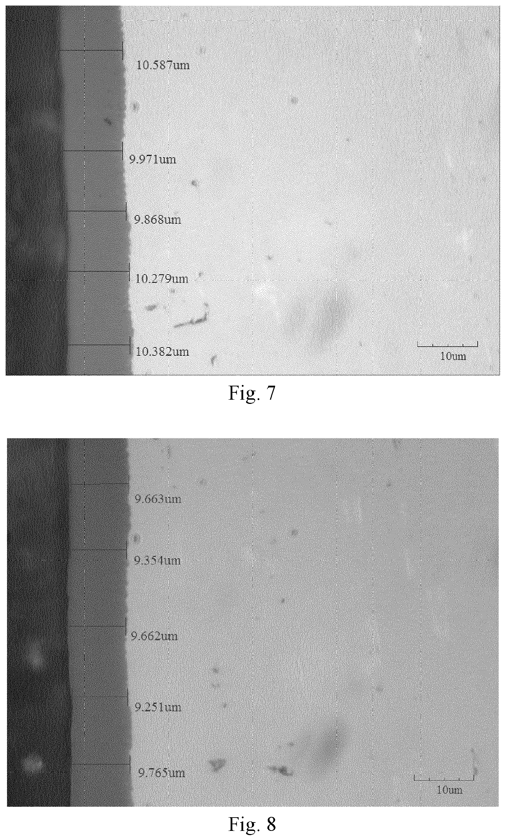

As shown in Fig. 7, after the oxidation treatment, Sample 4 had an oxidation ceramic surface layer with an average thickness of about 10.22 µm. While the thickness is out of the optimal range (3-7 µm), the sample surface remains to be flat. The oxidation ceramic surface layer is uniform in thickness and had a flat, clear interface with the substrate as well as good protective properties.

### Embodiment 3

Zr-2.5Nb alloy samples were ground and polished so that Sample 5 had a lowered surface roughness Ra of 0.0056 µm and Sample No. 6 had a lowered surface roughness Ra of 0.0055 µm. Samples 5 and 6 were placed into separate tube furnaces, and were both heated to 600 °C in air and maintained at the temperature for 4 h. Subsequently, for the furnace with Sample 5 placed therein, air was evacuated from and argon was introduced to the furnace following by the slow cooling at a rate of 5 °C/min to 100 °C or below. Then, Sample 5 was taken out from the furnace. By contrast, Sample 6 was slowly cooled still in the oxidizing atmosphere at a rate of 5 °C/min to 100 °C or below and then taken out. As measured with a roughness gauge, after the oxidation treatment, the roughness Ra of Sample 5 increased to 0.0162 µm and that of Sample 6 increased to 0.0426 µm.

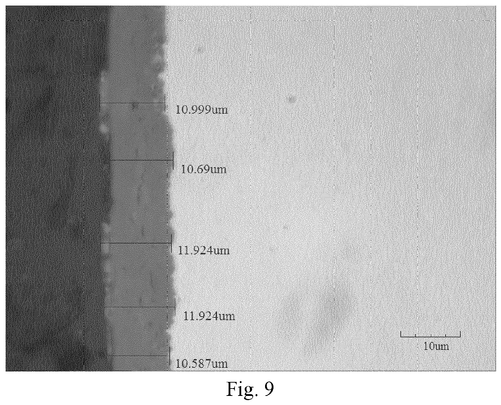

Fig. 8 is a metallurgical microscope image showing the cross section of Sample 5. Fig. 8 shows that the formed oxidation ceramic layer has a uniform average thickness of about 9.54 µm and a flat surface as well as a flat and straight interface with the substrate. Since the roughness Ra is within the range of Ra<0.02 µm, Sample 5 can be directly used in the fabrication of a bearing implant such as a hip or knee joints without further polishing. As Sample 6 is cooled in the initial oxidizing atmosphere, the surface of Sample 6 continues to be oxidized in the cooling process, so as to form a thicker oxidation ceramic surface layer. As shown in Fig. 9, the formed oxidation ceramic surface layer has a thickness of about 11.28 µm, and poor-quality oxides are present on the outer surface of the layer, which remarkably raises the surface roughness of Sample 6 that has been oxidized beyond the range of Ra<0.02 µm. Therefore, it has to be further polished before it can meet the application requirements of bearing implants such as hip and knee joints. Nonetheless, thanks to the polishing treatment prior to the oxidation treatment, the oxidation ceramic surface layer has a flat interface with the substrate, which allows the layer to maintain a uniform thickness after polishing and eliminate the problems of non-uniform thickness and excessive low thickness at some locations.

In summary, according to present application, roughness Ra of a zirconium/zirconium alloy surface has been lowered to Ra<0.01 µm before the surface oxidization treatment, allowing the oxidation ceramic surface layer to grows to the optimal thickness as well as enabling to control the roughness just within the required range (Ra<0.02 µm) of finished product. The cooling process in an inert atmosphere enables to avoid performance degradation of the oxidation ceramic surface layer during the cooling process, thereby enabling the grounding and polishing treatment after the oxidation treatment to be omitted to obtain an integral oxidation ceramic surface layer. In addition, as it is unnecessary to reserve a certain thickness for the loss arising from the grinding and polishing treatment, the oxidation ceramic surface layer is allowed to grow exactly to the optical thickness. This enables the oxide layer to have an optimal internal compactness, a high bonding strength and an excellent wear resistance.

It is to be noted that, the medical implants as mentioned herein refer to implantable medical instruments that can be placed into surgically created or naturally occurring cavities in human bodies. Examples of the medical implants may include, but are not limited to, surgical implants such as artificial joints, (orthopedic, spinal, cardiovascular and neurosurgical) implants, structural prostheses, dentures and other artificial organs; implants made of metal materials (including stainless steel, cobalt-based alloys, titanium and alloys thereof and shape memory alloys), polymers, high molecular materials, inorganic non-metallic materials, ceramic materials, etc.; implantable instruments such as implantable orthopedic instruments, implantable aesthetic and plastic surgical instrument and materials; implantable appliances such as bones (plates, screws, pins, rods), intra-spinal fixation devices, staplers, patellar concentrators, bone wax, bone repair materials, plastic surgical materials, heart or tissue repair materials, intraocular filling materials, nerve patch, etc.; interventional instruments such as interventional catheters, stents, embolization and other devices; and orthopedic (orthopedic) surgical instruments such as scalpels, drills, scissors, forceps, saws, chisels, files, hooks, needle slickers, active instruments, extremity extension braces, multi-purpose unilateral external fixation devices and other instruments for orthopedic (orthopedic) surgical use.

## Claims

1. A method for preparing a medical implant, comprising forming an oxidation ceramic layer on a surface of zirconium or a zirconium alloy, wherein the formation of the oxidation ceramic layer on a surface of zirconium or a zirconium alloy comprises steps of:
1) lowering a roughness of a targeted area of the zirconium or zirconium alloy on which a surface oxidization treatment is to be performed, the roughness being controlled to 0.01 µm or less;
2) performing the surface oxidation treatment by heating to a temperature in the range of 500°C to 700 °C and maintaining the temperature for 0.5-10 h in an atmosphere containing an oxidizing gas to form an oxidation ceramic surface layer; and
3) introducing an inactive gas to replace the oxidizing gas used in step 2) and initiating a cooling process to complete preparation of the oxidation ceramic surface layer without performing a subsequent treatment of lowering a roughness of the oxidation ceramic surface layer.

2. The method of claim 1, wherein the roughness is lowered to a value in the range of 0.002µm to 0.010 µm in step 1).

3. The method of claim 1, wherein after the oxidation treatment in step 2), a thickness of the oxidation ceramic surface layer on the targeted area is controlled within a range of 1µm to 20 µm.

4. The method of claim 1, wherein the roughness of the targeted area is lowered in step 1) by means of grinding, mechanical polishing, fine machining, vibratory polishing or any combination thereof.

5. The method of claim 1, wherein the inactive gas in step 3) is one or more selected from the group consisting of nitrogen and rare gases.

6. The method of claim 1, wherein the targeted area for the surface oxidization treatment is an area to be brought into direct contact with another bearing surface.

7. The method of claim 1, wherein the atmosphere containing the oxidizing gas in step 2) is an atmosphere containing oxygen and/or ozone.

## Patentansprüche

1. Verfahren zur Herstellung eines medizinischen Implantats, umfassend ein Bilden einer Oxidationskeramikschicht auf einer Oberfläche von Zirkonium oder einer Zirkoniumlegierung, wobei das Bilden der Oxidationskeramikschicht auf einer Oberfläche von Zirkonium oder einer Zirkoniumlegierung folgende Schritte umfasst:
1) Verringern einer Rauheit eines Zielbereichs des Zirkoniums oder der Zirkoniumlegierung, auf dem eine Oberflächenoxidationsbehandlung durchzuführen ist, wobei die Rauheit auf 0,01 µm oder weniger gesteuert wird;
2) Durchführen der Oberflächenoxidationsbehandlung durch Erhitzen auf eine Temperatur im Bereich von 500°C bis 700°C und Halten der Temperatur für 0,5-10 h in einer Atmosphäre, die ein oxidierendes Gas enthält, um eine Oxidationskeramikoberflächenschicht zu bilden; und
3) Einleiten eines inaktiven Gases, um das in Schritt 2) verwendete Oxidationsgas zu ersetzen, und Initiieren eines Abkühlungsprozesses, um eine Erstellung der Oxidationskeramikoberflächenschicht abzuschließen, ohne eine anschließende Behandlung zur Verringerung einer Rauheit der Oxidationskeramikoberflächenschicht durchzuführen.

2. Verfahren nach Anspruch 1, wobei in Schritt 1) die Rauheit auf einen Wert im Bereich von 0,002 µm bis 0,010 µm verringert wird.

3. Verfahren nach Anspruch 1, wobei nach der Oxidationsbehandlung in Schritt 2) eine Dicke der Oxidationskeramikoberflächenschicht auf dem Zielbereich in einem Bereich von 1 µm bis 20 µm gesteuert wird.

4. Verfahren nach Anspruch 1, wobei die Rauheit des Zielbereichs in Schritt 1) durch Schleifen, mechanisches Polieren, Feinbearbeitung, Vibrationspolieren oder eine beliebige Kombination davon verringert wird.

5. Verfahren nach Anspruch 1, wobei das inaktive Gas in Schritt 3) eines oder mehrere aus der Gruppe bestehend aus Stickstoff und Edelgasen ist.

6. Verfahren nach Anspruch 1, wobei der Zielbereich für die Oberflächenoxidationsbehandlung ein Bereich ist, der in direkten Kontakt mit einer anderen Lageroberfläche gebracht werden soll.

7. Verfahren nach Anspruch 1, wobei die Atmosphäre, die das oxidierende Gas in Schritt 2) enthält, eine Atmosphäre ist, die Sauerstoff und/oder Ozon enthält.

## Revendications

1. Procédé de préparation d'un implant médical, comprenant la formation d'une céramique d'oxydation couche sur une surface de zirconium ou d'un alliage de zirconium, la formation de la céramique d'oxydation couche sur une surface de zirconium ou d'un alliage de zirconium comprend les étapes consistant à:
1) abaisser une rugosité d'une zone ciblée du zirconium ou de l'alliage de zirconium sur laquelle un traitement d' oxydation de surface doit être effectué, la rugosité étant contrôlée à 0,01 µm ou moins;
2) effectuer le traitement d'oxydation de surface en chauffant à une température comprise entre 500°C et 700°C et maintien de la température pendant 0,5-10 h dans une atmosphère contenant un gaz oxydant pour former une couche superficielle de céramique d'oxydation; et
3) introduction d'un gaz inactif pour remplacer le gaz oxydant utilisé à l'étape 2) et initiation d'un processus de refroidissement pour achever la préparation de la couche superficielle de céramique d'oxydation sans effectuer un traitement ultérieur d'abaissement d'une rugosité de la couche superficielle de céramique d'oxydation.

2. Procédé selon la revendication 1, dans lequel la rugosité est abaissée à une valeur comprise entre 0,002 µm et 0,010 µm à l' étape 1).

3. Procédé selon la revendication 1, dans lequel après le traitement d'oxydation de l' étape 2), une épaisseur de la couche superficielle de céramique d'oxydation sur la zone ciblée est contrôlée dans une plage de 1 µm à 20 µm.

4. Procédé selon la revendication 1, dans lequel la rugosité de la zone ciblée est abaissée à l'étape 1) au moyen d'un meulage, d'un polissage mécanique, d'un usinage fin, d'un polissage vibratoire ou de toute combinaison de ceux-ci.

5. Procédé selon la revendication 1, dans lequel le gaz inactif à l'étape 3) est un ou plusieurs gaz choisis dans le groupe constitué d' azote et de gaz rares.

6. Procédé selon la revendication 1, dans lequel la zone ciblée pour le traitement d'oxydation de surface est une zone à mettre en contact direct avec une autre surface d'appui.

7. Procédé selon la revendication 1, dans lequel l' atmosphère contenant le gaz oxydant à l'étape 2) est une atmosphère contenant de l'oxygène et/ou de l'ozone.
